# EUROPEAN PATENT SPECIFICATION

(11) **EP 3 334 451 B1**
(45) Date of publication and mention of the grant of the patent: **28.10.2020**
(21) Application number: 16753893.3
(22) Date of filing: 12.08.2016
(51) Int. Cl.: A61K 38/30, A61K 31/728, A61K 31/727, A61K 31/726, A61K 31/738, A61K 38/17, A61K 9/00, A61P 19/02

(54) **COMPOSITION FOR THE TREATMENT OF JOINT CONDITIONS**
ZUSAMMENSETZUNG ZUM SCHUTZ VON POLYSACCHARIDEN IN GELENKEN
COMPOSITION POUR LA PROTECTION DE POLYSACCHARIDES DANS DES ARTICULATIONS

(30) Priority: 13.08.2015 EP 15180866
(43) Date of publication of application: 20.06.2018
(73) Proprietor: Stoffel, Bernard, 6043 Ransart (BE); Sipsma, Charles Henrij, 7491 CD Delden (NL); van der Tuin, Everhardus, 7491 CK Delden (NL); Humphrys, Simon Troy, Kangarilla, South Australia 5157 (AU); Newven sprl, 6043 Ransart (BE)
(72) Inventor: STOFFEL, Bernard, 6043 Ransart (BE); SIPSMA, Charles Henrij, 7491 CD Delden (NL); VAN DER TUIN, Everhardus, 7491 CK Delden (NL); HUMPHRYS, Simon Troy, Kangarilla, South Australia 5157 (AU)
(74) Representative: ABYOO
(86) International application number: PCT/EP2016/069279
(87) International publication number: WO 2017/025635

(56) References cited:
- WO-A1-00/37124
- WO-A1-87/01038
- WO-A2-99/51262
- WO-A2-03/000191
- FR-A1- 2 865 737
- MORALES TERESA I: "The quantitative and functional relation between insulin-like growth factor-I (IGF) and IGF-binding proteins during human osteoarthritis", JOURNAL OF ORTHOPAEDIC RESEARCH, vol. 26, no. 4, April 2008 (2008-04), pages 465-474, XP055276712, ISSN: 0736-0266
- ARAI T ET AL: "BINDING OF INSULIN-LIKE GROWTH FACTOR (IGF) I OR II TO IGF-BINDING PROTEIN-2 ENABLES IT TO BIND TO HEPARIN AND EXTRACELLULAR MATRIX", ENDOCRINOLOGY, THE ENDOCRINE SOCIETY, US, vol. 137, no. 11, 1 January 1996 (1996-01-01), pages 4571-4575, XP009055751, ISSN: 0013-7227, DOI: 10.1210/EN.137.11.4571
- FRANCIS G L ET AL: "INSULIN-LIKE GROWTH FACTOR (IGF)-II BINDING TO IGF-BINDING PROTEINS AND IGF RECEPTORS IS MODIFIED BY DELETION OF THE N-TERMINAL HEXAPEPTIDE OR SUBSTITUTION OF ARGININE FOR GLUTAMATE-6 IN IGF-II", BIOCHEMICAL JOURNAL, PORTLAND PRESS LTD, GB, vol. 293, no. 3, 1 January 1993 (1993-01-01), pages 713-719, XP008048625, ISSN: 0264-6021

## Description

### Field of the invention

The invention relates to a composition and methods for enhancing the intra-articular stability of polyscaccharides or derivatives thereof, and/or for prolonging the effects of intra-articular polysaccharides or derivatives thereof. The invention also relates to a composition and methods for treating articular joints in need thereof. The present invention further relates to a process for the preparation of said composition.

### Background of the invention

Healthy articular cartilage and synovial fluid are essential functional elements of articular joints that repeatedly dissipate biomechanical forces ranging from 3x body weight (walking) to 20x body weight (jumping). Mechanical stressors such as sheer forces and compression from weight bearing exercise provides a continual insult to the integrity and biomechanical functionality of synovial fluid and its capacity to promote healthy articular cartilage and synovium.

Articular cartilage and synovium are more or less susceptible to degenerative changes due to the balance between essential macromolecule synthesis, degradation and remodeling by chondrocytes, synoviocytes and immune cells. These cells regulate turnover of essential macromolecules via auto-paracrine growth factor and cytokine cascades. The resultant synovial fluid and cartilage maintains joint functionality.

Injuries to articular cartilage due to biomechanically inferior synovial fluid and or cartilage that cannot adequately dissipate physical stressors and the long-term sequelae of these injuries are a major cause of disability and medical or veterinary costs. Cartilage damage has been documented in up to 61.5% of arthroscopies performed for knee symptoms and about 41,000 surgical procedures to repair cartilaginous defects are performed annually in the United States. Joint problems are extremely common in domestic animals, especially dogs and horses. In the horse, lameness due to joint problems has been shown to be the commonest cause of either euthanasia or death in some equine populations. In horses and dogs used for fast athletic work, joint injury and subsequent osteoarthritis is extremely common, and is a major reason why horses/dogs are unable to compete or train. Obesity is also a major risk factor that causes extraordinary physical stressors which synovial fluid and articular cartilage must dissipate in articular joints in humans, and in companion animals such as dogs and cats.

A number of therapies for conditions in which articular cartilage has already suffered damage, such as tears or osteoarthritis, are available. They are either directed at providing symptomatic relief by reducing inflammation (Steroids and NSAIDs), by ensuring ample dietary supply of joint synovial fluid/articular cartilage components, by replacing synovial fluid with polysaccharide formulations and more recently by conditioning synovial fluid and cartilage using autologous cell transplant therapies. Anti-inflammatories (irrespective of route of administration) are effective at reducing the symptoms of joint damage but don't treat the underlying pathologies, damage or conditions and don't promote repair of joint structures. Anti-inflammatories also need to be administered repeatedly to maintain any clinical effect and are banned as performance enhancing in competing humans, horses, and dogs.

Formulated polysaccharide treatments are limited to oral and injectable formulations of glucosamine (GN), chondroitin sulphate (CS), polysulphated carbohydrates (pentosan polysulphate sodium - PPS) or hyaluronic acid (HA), which are marketed as treatments which will provide the joint with readily available cartilage matrix components or which provide viscosupplementation to joints.

Orally administered polysaccharide therapies need to be taken daily and demonstrate the weakest clinical effect. Injectable formulations administered via intravenous, intramuscular or intra-articular routes often vary in their duration of effect and range from 1-12 weeks. This necessitates repeated administrations, which carries the risk of physical damage to the joint or infection.

Intra-articular injection is required for all viscosupplementation treatments, with higher molecular mass products generally considered to result in a superior clinical effect for longer, but the degree of effect, strength of correlation and safety of increasingly higher molecular mass HA has not been demonstrated in clinical studies in humans, horses or dogs.

The document WO 00/37124 discloses compositions comprising cross-linked hyaluronic acid for the treatment of cartilage damage, said compositions possibly further comprising IGF.

Autologous cell transplant therapies are a recent phenomena where cells are removed from an animal and re-programmed using cell signaling peptides/chemicals to achieve a specific phenotype eg HA production for better synovial fluid or cartilage component production eg collagen type II. The benefits of these treatments are entirely dependent on the longevity of the components that the autologous cells have been programed to synthesize.

Recent studies have demonstrated that hyaluronic acid breakdown products or fragments can have a deleterious effect on articular cartilage so new ways of stabilizing or prolonging the benefits of HA injected into joints will reduce physiological insults to cartilage/synovium and prolong the viscosupplementation effect and clinical benefits of this treatment.

There is therefore a need for improved compositions used in viscosupplementation of articular joints.

### Summary of the invention

In a first aspect of the present invention, a composition is provided. Said composition comprises:
a) hyaluronic acid as a compound (A), cross-linked derivatives thereof, and/or autologous cells reprogrammed to synthetize and secrete a compound (A), and
b) des(1-3)-IGF-I as an analogue of mammalian insulin-growth factor-1 wherein from 1 to 5 amino acid residues are absent from the N-terminal or an amino acid substitution is made to native amino acid residues 1-5.

An unexpected increase in duration of clinical effect was observed in horses treated with intraarticular injections of such a composition according to the present invention. Relevant prior art teaches away from this effect because the analogue has a very short half-life in mammals, of <30 minutes (Ballard, 1996), and the analogue does not reduce proteoglycan catabolism (Morales, 2007). Horses injected with this combination were surprisingly clinically normal for significantly longer than horses injected with hyaluronic acid alone. A composition according to the first embodiment of the invention may also significantly prolong and/or enhance the clinical effects of hyaluronic acid viscosupplementation within joints.

In a preferred embodiment, the compound (A) is a cross-linked compound. In a more preferred embodiment, the compound (A) is at least partly DVS (divinylsulfone) crosslinked with des(1-3)-IGF-I as the analogue of mammalian IGF-1.

In a second aspect of the present invention, a composition for the treatment or prophylaxis of articular cartilage conditions or diseases is provided. Said composition comprises:
a) hyaluronic acid as a compound (A) and/or autologous cells reprogrammed to synthetize and secrete a compound (A), and
b) des(1-3)-IGF-I as an analogue of mammalian insulin-growth factor-1 wherein from 1 to 5 amino acid residues are absent from the N-terminal or an amino acid substitution is made to native amino acid residues 1-5.

In a third aspect of the present invention, a method for the preparation of the compositions according to the present invention is provided. Said process comprises the steps of:
(a) reacting divinyl sulphone with a compound (A) at a pH ranging from 8 to 11 using a divinyl sulphone to compound (A) molar ratio between 1 and 10%; and
(b) adding des(1-3)-IGF-I as an analogue of mammalian insulin-growth factor-1 to a maximal concentration of analogue of 1mg per milliliter of solution obtained in step (a).

It was surprisingly found that said analogue of mammalian insulin-growth factor-1 according to the present invention can increase the duration of the viscosupplementation effect of compound (A), in the joint once administered. Moreover, it was surprisingly found that said analogue of mammalian insulin-growth factor-1 according to the present invention can increase the duration of the lifetime of compound (A) in the joint administered. This has been particularly observed with cross-linked hyaluronic acid as compound (A). The breakdown products of compound (A) or fragments thereof have been shown to adversely affect chondrocyte and synoviocyte metabolism and health. Despite the very short half-life of the analogue of mammalian insulin-growth factor-1 it unexpectedly prolonged the time that compound (A) is clinically beneficial to the joint after administration. Thus, this product is superior to currently available products and reduces the frequency of repeat intra-articular injections and the pain/risks associated with this route of administration or the need for more invasive therapies.

### Detailed description of the invention

For the purposes of this specification it will be clearly understood that the word "comprising" means "including but not limited to", and that the word "comprises" has a corresponding meaning.

A synovial joint is a freely movable joint in which contiguous bony surfaces are covered by hyaline cartilage (articular cartilage), and connected by a fibrous connective tissue capsule lined with synovial membrane; synovial fluid, which lubricates and protects the articular cartilage, is contained within the synovial cavity which is surrounded by the synovial membrane and joint capsule. Synovial joints include the hip, knee, elbow, shoulder, ankle and wrist.

The "joint" means the whole synovial joint, ie the joint capsule, synovial membrane, synovial fluid, articular cartilage, and accessory structures such as the meniscus in the knee joint. "Conditioning synovial fluid" means modifying its biomechanical properties by enhancing the stability and protective effect of viscosupplementation, which in turn reduces the risk of damage to articular joints.

Adverse effects of intensive weight-bearing exercise on joints include but are not limited to increased risk of trauma, increased frictional stress on cartilage, impact-load injury, long-term damage caused by wear and tear, and increased risk of osteoarthritis.

The insulin-like growth factors (IGFs) are proteins which have high sequence similarity to insulin. They are part of a complex system known as the IGF "axis" via which cells communicate with their physiological environment. IGF-I is mainly secreted by the liver as a result of stimulation by growth hormone but is also secreted by a variety of cell types, including synoviocytes and chondrocytes in joints, in order to stimulate autocrine/paracrine activities. The IGF axis has been shown to be involved in the promotion of cell proliferation and the inhibition of cell death (apoptosis). Factors known to cause variation in the levels of growth hormone and IGF-I in the circulation include ethnicity, individual genetic make-up, time of day, age, sex, exercise status, stress levels, genetics, nutrition level and body mass index, disease state, oestrogen status and xenobiotic intake.

The terms "fragment", "analogue", and "derivative" of IGF refer to a molecule which retains some elements and loses some elements of the same biological function or activity as IGF, i.e. analogues or derivatives of human IGF in which the wild-type IGF sequence includes additions, deletions or substitutions by another amino acid or an amino acid analogue, provided that the biological activity of the IGF is retained. Thus an analogue includes a pro-protein which can be activated by cleavage of the pro-protein portion to produce an active mature polypeptide.

An analogue of insulin-growth factor-1 is a fragment, analogue or derivative of IGF-I or a chemical mimic of IGF-I which has an equivalent or higher capacity to bind to and stimulate the IGF-I type I receptor and a diminished capacity to bind IGF-binding proteins (IGFBPs) compared to that of native IGF-I. IGF agents suitable for use in this invention may readily be identified using methods known in the art. For example, the capacity of a given candidate molecule to bind to and stimulate the IGF-I type I receptor may be measured by the method of Forbes et al. (2002). The capacity of a given candidate molecule to bind IGFBPs may be measured by the method of Szabo et al. (1988).

Des(1-3)IGF-I is a naturally-occurring IGF-I variant, a truncated form of IGF-I in which the N-terminal tripeptide, glycine-proline-glutamine is absent. It is found in tissues and biological fluids, probably as a result of post-translational protease cleavage of the sequence of circulating mature IGF-I. This will reduce the probability that it is immunogenic and generate an inflammatory response once injected into joints. Des(1-3)IGF-I is approximately 5-10 times more potent than IGF-I in most but not all in vitro assays, for example it stimulates protein or DNA synthesis and inhibits protein breakdown at concentrations between 10% and 20% of those required with IGF-I (Francis et al., 1988a, 1988b; Ballard et al., 1987). The increased potency results from a lower affinity to IGFBPs, which also increases its clearance rate from the body (Ballard et al., 1996). Therefore the capacity of des(1-3)IGF-I to significantly increase the duration of a clinical effect induced by single or infrequent intraarticular administration is unexpected. In chondrocytes, des(1-3)IGF-I is more potent at lower concentrations than IGF-I in stimulating incorporation of newly synthesized proteoglycans into the cell layer; 3-fold with des(1-3)IGF-I compared to 2-fold for IGF-I (Sunic et al., 1995). Des(1-3)IGF-I was also more effective than IGF-I at stimulating proteoglycan synthesis in cultured cartilage slices, but was ineffective at reducing proteoglycan catabolism (Morales, 2007), a key process in the degeneration of joint structures. Despite its effects *in vitro* no *in vivo* studies have assessed the efficacy of des(1-3)IGF-I in prolonging the biomechanical functionality of said compounds (A) and duration of viscosupplementation effect. Moreover, until now, no studies have assessed the efficacy of des(1-3)IGF-I in prolonging the duration of effect for viscosupplementation treatments *in vivo.*

R³IGF-I is a recombinant analogue of human IGF-I in which there is a substitution of Arg for Glu₃. R³IGF-I binds more weakly to IGFBPs than IGF-I (Milner S.J. (2004) GroPep Bioreagents Technical Bulletin No. 2).

Insulin-like Growth Factor Binding Proteins (IGFBPs) are a group of secreted proteins found in vertebrates, which bind to IGF-I and IGF-II with high affinity and modulate the biological actions of IGFs. The IGFBP family has six distinct subgroups, IGFBP-1 through 6, based on conservation of gene (intron-exon) organisation, structural similarity, and binding affinity for IGFs. All IGFBPs share a common domain architecture. While the N-terminal (IGF binding protein domain), and the C-terminal (thyroglobulin type-1 repeat) domains are conserved across vertebrate species, the mid-region is highly variable with respect to protease cleavage sites and phosphorylation and glycosylation sites.

IGFBPs can prolong the half-life of IGFs and also serve to regulate the endocrine and paracrine/autocrine actions of IGF by modulating the amount of IGF available to bind to signaling IGF-I receptors.

A polysaccharide is any member of a class of carbohydrates, also called glycans, whose molecules consist of a number of monosaccharides joined by glycosidic bonds. Polysaccharides include starch and cellulose.

Glycosaminoglycans (GAGs) or mucopolysaccharides are linear polysaccharides, made up of repeating disaccharide units consisting of an amino sugar (either N-acetyl glucosamine or N-acetyl galactosamine) and an uronic acid (glucuronic acid or iduronic acid). They are highly negatively charged because of the presence of uronic acid and sulphate groups, and have an extended conformation which confers high viscosity. GAGs are prominent in the extracellular matrix, and include hyaluronan (hyaluronic acid), dermatan sulphate, chondroitin sulphate, heparin, heparan sulphate, and keratan sulphate. Most naturally-occurring GAGs are polysulphated. Either polysulphated or unsulphated GAGs may be used.

Hyaluronic acid (hyaluronan) is the only GAG which does not contain any sulphate and is not found covalently attached to proteins as a proteoglycan. However, it is a component of non-covalently formed complexes with proteoglycans in the extracellular matrix. Hyaluronic acid polymers are very large, with molecular weights of 100,000-10,000,000, and can displace a large volume of water.

Proteoglycans are heavily glycosylated proteins which consist of a core protein with one or more GAG molecules covalently attached to serine residues in the protein via a tetrasaccharide bridge. Proteoglycans are important components of connective tissue, such as the matrix of articular cartilage.

Glycoproteins are proteins in which one or more oligosaccharide chains (glycans) are covalently attached to side-chains of amino acid residues in the protein.

An "autologous cell" as used herein is a cell (stem cell, undifferentiated cell, induced pluripotent stem cell (iPSC), somatic cell) removed from a person or an animal, and later given back to the same person or animal. Removed cells may be reprogrammed for expressing and secreting joint macromolecules through the introduction of exogenous factors, like OSKM factors (Oct4/Sox2/Klf4/ c-Myc), cytokines, growth factors, transcription factor(s), expression vector(s) like a viral vector such as a retrovirus, before re-implanting or administering back the desired phenotypically differentiated cell to the same person or animal. For example, an autologous cell may be a stem cell, iPSC, somatic cell, a synoviocyte cell, a chondrocyte cell, a fibroblast cell, an endothelial cell, an epithelial cell transfected by a plasmid expression vector, a baculovirus expression vector, an adenoviral expression vector, coding for any one of the compounds (A) selected from the group polysulphated and unsulphated polysaccharides, polysulphated and unsulphated glycosaminoglycans, polysulphated and unsulphated proteoglycans, and polysulphated and unsulphated glycoproteins.

The term "effective amount" as used herein means that amount necessary at least partly to attain the desired effect, i.e. protection of the articular cartilage of the joint from the adverse effects of intensive weight-bearing exercise. Such amounts will depend on the characteristics of the individual subject, including species, age, physical condition, size, weight, whether there has been a prior injury, and whether the subject is receiving any other concurrent treatment, and will be at the discretion of the attending physician or veterinarian. These factors are well known to those of ordinary skill in the art, and can be addressed with no more than routine experimentation. It is generally preferred that a minimum effective dose be determined according to sound medical or veterinary judgement. It will be understood by those of ordinary skill in the art that a higher dose may be administered for medical, psychological or other reasons.

As mentioned above, the present invention relates to a composition which is suitable to prolong the stability of a type of polysaccharide or polysaccharide derivatives, therefore allowing an enhanced treatment or enhanced prophylaxis of articular joint conditions and or diseases.

The present composition comprises:
(a) hyaluronic acid as a compound (A) or salts thereof, and/or autologous cells reprogrammed to synthetize and secrete a compound (A), and
(b) des(1-3)-IGF-I as an analogue of mammalian insulin-growth factor-1 wherein from 1 to 5 amino acid residues are absent from the N-terminal or an amino acid substitution is made to native amino acid residues 1-5.

In an alternative embodiment, the present composition comprises autologous cells reprogrammed to synthetize and secrete hyaluronic acid as a compound (A) or salts thereof and des(1-3)-IGF-I as an analogue of mammalian insulin-growth factor-1 wherein from 1 to 5 amino acid residues are absent from the N-terminal or wherein from 1 to 5 amino acid residues are added at the N-terminal.

In a preferred embodiment, said compound (A) can be cross-linked. In a more preferred embodiment, the compound (A) is at least partly DVS cross-linked with des(1-3)-IGF-I as the analogue of IGF-1.

In particular, said compound (A) may bear divinylsulfone moieties of formula -[CR¹-CR²-SO₂-CR³-CR⁴]- wherein R¹, R², R³ and R⁴ are independently selected from the group consisting of hydrogen, C₁-C₁₀ alkyl, C₆-C₁₂ aryl, C₃-C₁₀ cycloalkyl, or R¹, R² or R³, R⁴ form together with the carbon atom to which they are linked a C₆-C₁₂ aryl substituted or not by C₁-C₁₀ alkyl or C₂-C₁₀ alkene. Particularly, said compound (A) may bear divinylsulfone moieties of formula -CH₂-CH₂-SO₂-CH₂-CH₂-.

In a preferred embodiment, said compound (A) has a molecular weight greater than 0.1 MDa, preferably greater than 0.5 MDa, more preferably greater than 0.7 MDa. In particular, said compound (A) may have a molecular weight ranging from 0.7 to 8 MDa, preferably ranging from 1.2 to 5 MDa.

In a preferred embodiment, said compound (A) is a cross-linked compound or salts thereof having a molecular weight ranging from 0.7 to 8 MDa, preferably ranging from 1.2 to 5 MDa.

In a preferred embodiment, said compound (A) is a liquid form. Preferably said compound (A) may have a viscosity greater than 5 Pa.s, preferably greater than 10 Pa.s. In a more preferred embodiment, said compound (A) may have a viscosity ranging from 15 Pa.s to 40 Pa.s as measured according to the Brookfield test method, preferably from 18 Pa.s to 32 Pa.s.

Preferably, said compound (A) may be a liquid form and may have a viscosity greater than 5 Pa.s, preferably greater than 10 Pa.s, more preferably ranging from 15 Pa.s to 40 Pa.s, most preferably ranging from 18 Pa.s to 32 Pa.s as measured according to the Brookfield test method; and having a molecular weight greater than 0.1 MDa, preferably greater than 0.5 MDa, more preferably greater than 0.7 MDa, most preferably ranging from 0.7 to 8 MDa, even most preferably ranging from 1.2 to 5 MDa.

In particular, said compound (A) is a liquid and cross-linked compound or salts thereof having a viscosity greater than 5 Pa.s, preferably greater than 10 Pa.s, more preferably ranging from 15 Pa.s to 40 Pa.s, most preferably ranging from 18 Pa.s to 32 Pa.s as measured according to the Brookfield test method; and having a molecular weight greater than 0.1 MDa, preferably greater than 0.5 MDa, more preferably greater than 0.7 MDa, most preferably ranging from 0.7 to 8 MDa, even most preferably ranging from 1.2 to 5 MDa.

In a more particular embodiment, said compound (A) is a solution of hyaluronic acid or salts thereof having a viscosity greater than 5 Pa.s, preferably greater than 10 Pa.s, more preferably ranging from 15 Pa.s to 40 Pa.s, most preferably ranging from 18 Pa.s to 32 Pa.s as measured according to the Brookfield test method; and having a molecular weight greater than 0.1 MDa, preferably greater than 0.5 MDa, more preferably greater than 0.7 MDa, most preferably ranging from 0.7 to 8 MDa, even most preferably ranging from 1.2 to 5 MDa. Preferably the solution is an aqueous solution.

Preferably, in the present composition, the effective amount of compound (A) is greater than 0.001wt%, preferably greater than 0.005wt%, more preferably greater than 0.01 wt%, and is at most 20wt%, preferably at most 15wt%, more preferably at most 10wt%. In a preferred embodiment, the effective amount of compound (A) ranges from 0.01 wt% to 10 wt% based on the total weight of the composition, preferably from 0.01 wt% to 8wt%, more preferably from 0.01 wt% to 5wt%, even more preferably from 0.05wt% to 5 wt%, most preferably from 0.1 wt% to 5 wt%, even most preferably from 0.5 wt% to 3wt%.

In a preferred embodiment, the analogue of mammalian insulin-growth factor-1 has from 1 to 10 amino acid residues absent from the N-terminal, preferably from 1 to 5 amino acid residues absent from the N-terminal.

Preferably, the analogue is an equine, canine and or human insulin-like growth factor-1 analogue.

More preferably, the analogue has an N-terminal structure selected from
Pro-glu-thr-leu-cys-gly-ala-glu-leu-val-asp-ala-leu-gln-phe-val-cys-gly-asp-arg-gly-phe-tyr-phe-asn-lys-pro-thr-gly-, noted SEQ1
Glu-thr-leu-cys-gly-ala-glu-leu-val-asp-ala-leu-gln-phe-val-cys-gly-asp-arg-gly-phe-tyr-phe-asn-lys-pro-thr-gly-, noted SEQ2
Thr-leu-cys-gly-ala-glu-leu-val-asp-ala-leu-gln-phe-val-cys-gly-asp-arg-gly-phe-tyr-phe-asn-lys-pro-thr-gly-, noted SEQ3
Leu-cys-gly-ala-glu-leu-val-asp-ala-leu-gln-phe-val-cys-gly-asp-arg-gly-phe-tyr-phe-asn-lys-pro-thr-gly-, noted SEQ4 or
Cys-gly-ala-glu-leu-val-asp-ala-leu-gln-phe-val-cys-gly-asp-arg-gly-phe-tyr-phe-asn-lys-pro-thr-gly-, noted SEQ5.

When the composition comprises reprogrammed autologous cells, an expression vector transfected inside these cells comprise a coding sequence for an IFG-1 analogue in a suitable site for the insertion of a gene such as the multiple cloning site of an expression vector. The coding sequence furthermore comprises any one of SEQ1, SEQ2, SEQ3, SEQ4 or SEQ5 has an N-terminal sequence.

According to the invention, the analogue is des(1-3)-IGF-I. Said des(1-3)-IGF-I may have an N-terminal structure of the following sequence thr-leu-cys-gly-ala-glu-leu-val-asp-ala-leu-gln-phe-val-cys-gly-asp-arg-gly-phe-tyr-phe-asn-lys-pro-thr-gly-.

When the composition comprises reprogrammed autologous cells, the expression vector transfected inside these cells leads to the expression of the analogue des(1-3)-IGF-I, eventually having an N-terminal structure of the following sequence thr-leu-cys-gly-ala-glu-leu-val-asp-ala-leu-gln-phe-val-cys-gly-asp-arg-gly-phe-tyr-phe-asn-lys-pro-thr-gly-.

The compositions according to the present invention can be used as a medicine. In a preferred embodiment, the compositions according to the present invention are suitable for the treatment or prophylaxis of conditions and or diseases affecting articular joints.

In a preferred embodiment, the effective amount of said analogue is greater than 1 µg/ml, preferably greater than 5 µg/ml, more preferably greater than 10 µg/ml, even more preferably greater than 30 µg/ml, and at most 300 µg/ml, preferably at most 200 µg/ml, more preferably at most 150 µg/ml, even more preferably at most 100 µg/ml, most preferably at most 75 µg/ml based on the total weight of the composition. In a preferred embodiment, the effective amount of said analogue may range from 15 µg/ml to 150 µg/ml, preferably from 20 µg/ml to 100 µg/ml, more preferably from 30 µg/ml to 60 µg/ml based on the total weight of the composition.

In a preferred embodiment, said composition is administered in a joint one to three times during the first month of treatment and one to 2 times per 3-6 months thereafter.

The present invention provides an efficient solution to limit the degradation of compound (A) and/or prolong the effect of compound (A) being hyaluronic acid. Adding the analogue according to the present invention may also allow the avoidance of hyaluronic acid's breakdown, in particular when both compounds are administered simultaneously or sequentially in the joint of a human or an animal.

Hence, said analogue of mammalian insulin-growth factor-1 wherein from 1 to 5 amino acid residues are absent from the N-terminal or wherein substitutions to native amino acids 1-5 are made can be used to increase the stability and/or the duration of viscosupplementation effect of a liquid composition of the cross-linked or not compound (A) or salts thereof. According to the invention, said analogue of mammalian insulin-growth factor-1 wherein from 1 to 5 amino acid residues are absent from the N-terminal, being des(1-3)-IGF-1, can be used to increase the stability of a liquid composition of the cross-linked compound (A) or salts thereof, compound (A) being hyaluronic acid or salts thereof as disclosed above.

As disclosed above, the composition according to the present invention may comprise a compound (A) which is cross-linked according to a particular embodiment of the present invention. Said cross-linked compound (A) may be prepared by a process comprising the step of reacting a sulfone compound with a compound (A) or salts thereof. Preferably, said step may be carried out at a pH ranging from 7 to 11, preferably from 8 to 9. Preferably, said step may be carried out at a sulfone compound to compound (A) molar ratio between 0.5 and 15%, preferably between 1 and 10%, more preferably between 2 and 5%. In a preferred embodiment, the sulfone compound is of formula -[CR¹=CR²-SO₂CR³=CR⁴]- wherein R¹, R², R³ and R⁴ are independently selected from the group consisting of hydrogen, C₁-C₁₀ alkyl, C₆-C₁₂ aryl, C₃-C₁₀ cycloalkyl, or R¹, R² or R³, R⁴ form together with the carbon atom to which they are linked a C₆-Cₗ₂ aryl substituted or not by C₁-C₁₀ alkyl or C₂-C₁₀ alkene. Particularly, said sulfone compound is divinylsulfone. Said compound (A) is a cross-linked or not hyaluronic acid or salts thereof. According to the invention, the compound (A) is hyaluronic acid or salts thereof and the sulfone compound is divinyl sulfone, the weight ratio of hyaluronic acid or salts thereof (based on the sodium salt) to divinyl sulfone should be between 20 and 200, preferably between 40 and 120. A weight ratio hyaluronic acid / divinyl sulfone (WR _{hyaluronic acid/divinyl sulfone}) of 20 corresponds to a molar percentage of divinyl sulfone to hyaluronic acid (MP_{divinyl sulfone/hyaluronic acid}) of 17% (on the basis of an anhydro[sodium glucuronate - N-acetylglucosamine] disaccharide) or 8.5% (on average anhydromonosaccharide basis). Thus, the molar percentage of divinyl sulfone to hyaluronic acid (monosaccharide basis) should be between about 0.9 and 8.5, preferably between about 1.7 and 5. The solution to be crosslinked may contain other biopolymers (polysaccharides, proteins, glycoproteins etc.) in addition to hyaluronic acid, but preferably less than one third of the amount of hyaluronic acid, most preferably less than 5% thereof.

Hence, the present invention provides a process for the preparation of a composition according to the present invention comprising the steps of:
(a) reacting a sulfone compound as disclosed above with a compound (A) or salts thereof, at a pH ranging from 7 to 11 using a sulfone compound to compound (A) molar ratio between 1 and 10%;
(b) adding des(1-3)-IGF-I as an analogue of mammalian insulin-growth factor-1 wherein from 1 to 5 amino acid residues are absent from the N-terminal or an amino acid substitution is made to native amino acid residues 1-5.

In a preferred embodiment, the present process comprises the steps of:
(a) reacting divinyl sulfone with a compound (A) or salts thereof, at a pH ranging from 7 to 11 using a divinyl sulfone to compound (A) molar ratio between 1 and 10%;
(b) adding des(1-3)-IGF-I as an analogue of mammalian insulin-growth factor-1 wherein from 1 to 5 amino acid residues are absent from the N-terminal or an amino acid substitution is made to native amino acid residues 1-5.

Said compound (A) is hyaluronic acid or salts thereof.

Preferably, step (a) is carried out at a pH ranging from 8 to 9. Preferably, the divinyl sulfone to compound (A) molar ratio is between 2 and 5%.

Preferably, the present process comprises the steps of:
(a) reacting divinyl sulfone with hyaluronic acid or salts thereof, at a pH ranging from 8 to 9 using a divinyl sulfone to compound (A) molar ratio between 1 and 10%, preferably between 2 and 5%;
(b) adding des(1-3)-IGF-I as an analogue of mammalian insulin-growth factor-1 wherein from 1 to 5 amino acid residues are absent from the N-terminal.

In a preferred embodiment, steps (a) and (b) are carried out simultaneously at a pH ranging from 7 to 11, preferably from 8 to 9. Hence, the cross linking of hyaluronic acid or salts thereof, in presence of a sulfone compound, in particular divinyl sulfone, as disclosed above may carried out in presence of des(1-3)-IGF-I as said analogue of mammalian insulin-growth factor-1 .

In a preferred embodiment, step (a) of the process is carried out at a temperature ranging from -5°C to 25°C, preferably from 0°C to 20°C, more preferably from 0°C to 15°C, most preferably from 0°C to 12°C.

### Examples

20grams of hyaluronic acid (Sigma, *Streptococcus zooepidemicus*) was added to 1000ml of sterile water and stirred continually for 4-5 days until a 2% homogeneous solution resulted. The pH was adjusted to 11 and 15 mg of DVS was added (WR_{HA/DVS}= 53). The mixture was stirred continuously to homogeneity (2 days). The solution was then adjusted to pH 7 using

acetic acid and sterilized by filtration or heat treatment. This comparative solution was compared to a composition according to the present invention wherein to 2 ml of the hyaluronic acid solution so-obtained, 30 µg of des(1-3)-IGF-I was added. The comparative composition (without des(1-3)-IGF-I) and the composition according to the present invention was injected into the joints of horses to assess the efficiency thereof.

The efficiency of the composition according to the present invention was evaluated by visual diagnosis before and after a flexion test. A flexion test is a preliminary veterinary procedure performed on a horse. The purpose is to accentuate any pain that may be associated with a joint or soft-tissue structure, allowing the localization of a lameness to a specific area. The animal's leg is held in a flexed position for 30 seconds to up to 3 minutes, preferably 30 seconds, and then the horse is immediately trotted off and its gait is analyzed for abnormalities and unevenness. Flexions stretch the joint capsule, increase intra-articular and subchondral bone pressure, and compress surrounding soft tissue structures, which accentuates any pain associated with these structures.

An increase in lameness following a flexion test suggests that those joints or surrounding soft tissue structures may be a source of pain for the horse. The horse may take a few uneven steps, or may be lame for several minutes following the procedure. Flexion tests are considered positive if lameness is increased, although usually lameness is forgiven for the first few steps following flexion. The horse's response should be graded with each flexion and recorded. This allows comparison in lameness when rechecking after treatment has been implemented.

The degree of joint pain and mobility was classified into 5 groups according to the following test method:

| | |
|---|---|
| Group I: | no visible signs of lameness before flexion-test, after flexion-test lame but not more than 10 meters in trot, then lameness disappears. |
| Group II: | no visible signs of lameness before flexion-test, after flexion-test persistent lame on trot. |
| Group III: | visible signs of lameness before flexion-test, becomes worse after flexion-test but resolves to the original level within 10 meters of trot. |
| Group IV: | visible signs of lameness before flexion-test, after flexion-test persistent lame on trot. |
| Group V: | visible signs of lameness before flexion-test, after flexion-test not able to use the flexed leg anymore for at least 10 meters. |

Two groups having 30 horses each were formed. 15 out of 30 horses received the composition of the present invention while the others received the comparative composition. Three injections were performed at T0, T10 and T21 corresponding to day 0, day 10 and day 21. The final diagnosis was performed T168, i.e. 168 days after the first injection. The comparative composition affords a mean scoring of 3.64 +/- 0.67. The composition according to the present invention affords a mean scoring of 0.33 +/- 0.49. Hence, the composition according to the present invention wherein hyaluronic acid is stabilized by des(1-3)-IGF-I was demonstrated to be more effective than a composition wherein hyaluronic acid is not stabilized therewith.

## Claims

1. A composition comprising:
a) hyaluronic acid as a compound (A) or salts thereof, and/or autologous cells reprogrammed to synthetize and secrete a compound (A), and
b) des(1-3)-IGF-I as an analogue of mammalian insulin-growth factor-1 having from 1 to 5 amino acid residues absent from the N-terminal .

2. A composition according to the previous claim wherein the hyaluronic acid as compound (A) is a cross-linked compound having a molecular weight between 0.7 and 8 MDa, preferably between 1.2 and 5 MDa.

3. A composition according to any of the previous claims 1 or 2 wherein said composition is under a liquid form, and has a viscosity ranging from 15 Pa.s to 40 Pa.s as measured according to the Brookfield test method, preferably from 18 Pa.s to 32 Pa.s.

4. A composition according to any of the previous claim, wherein hyaluronic acid as the compound (A) is at least partly DVS cross-linked together with des(1-3)-IGF-I as an analogue of mammalian insulin-growth factor-1 having from 1 to 5 amino acid residues absent from the N-terminal.

5. A composition for use in the treatment of disease affecting articular cartilage comprising:
(a) hyaluronic acid as a compound (A) or salts thereof, and/or autologous cells reprogrammed to synthetize and secrete a compound (A), and
(b) des(1-3)-IGF-I as an analogue of mammalian insulin-growth factor-1 having from 1 to 5 amino acid residues absent from the N-terminal.

6. A composition for use according to claim 5 wherein the amount of hyaluronic acid as compound (A) in said composition ranges from 0.01 wt% to 5 wt% based on the total weight of the composition.

7. A composition for use according to any of the previous claims 5 or 6 wherein the amount of the analogue is greater than 10 µg/ml, preferably is greater than 30 µg/ml, more preferably ranges from 30 µg/ml to 60 µg/ml based on the total weight of the composition.

8. A composition for use according to any of the previous claims 5 to 7, wherein hyaluronic acid as the compound (A) is at least partly DVS cross-linked together with des(1-3)-IGF-I as an analogue of mammalian insulin-growth factor-1 having from 1 to 5 amino acid residues absent from the N-terminal.

9. A composition for use according to any of the previous claims 5 to 8, which is administered in a joint one to four times the first month of treatment and one per 6 months thereafter.

10. Composition according to any of the previous claims 5 to 9 for use in a method for limiting hyaluronic acid from breakdown and prolonging the viscosupplementation effect of hyaluronic acid intraarticular injections in the joint of a human or an animal comprising the steps of administering the composition.

11. Use of des(1-3)-IGF-I as an analogue of mammalian insulin-growth factor-1 wherein from 1 to 5 amino acid residues are absent from the N-terminal to increase the stability in a liquid composition of hyaluronic acid or salts thereof.

12. Process for the preparation of a composition according to any of the previous claims 1 to 4, comprising the steps of:
(a) reacting divinyl sulphone with hyaluronic acid as the compound (A), at a pH ranging from 8 to 11 using a divinyl sulphone to compound (A) molar ratio between 1 and 10%;
b) adding des(1-3)-IGF-I as an analogue of mammalian insulin-growth factor-1 wherein amino acid residues are modified, removed or added that maintain or enhance insulin-like growth factor type 1 receptor avidity and affinity and wherein amino acid residues are modified, removed or added that reduce insulin-like growth factor binding protein avidity and affinity.

13. Process according to the previous claim wherein steps (a) and (b) are carried out simultaneously at a pH ranging from 8 to 9.

## Patentansprüche

1. Zusammensetzung, umfassend:
a) Hyaluronsäure als ein Bestandteil (A) oder Salze davon und / oder autologe Zellen, die zum Synthetisieren und Sekretieren eines Bestandteils (A) neu programmiert sind, und
b) Des(1-3)-IGF-I als ein analoger Säugetier-Insulinwachstumsfaktor-1, der 1 bis 5 Aminosäure-Reste aufweist, die bei der N-Endung fehlen.

2. Zusammensetzung gemäß einem der voranstehenden Ansprüche, wobei die Hyaluronsäure als Bestandteil (A) ein vernetzter Bestandteil ist, der ein Molekulargewicht zwischen 0,7 und 8 MDa, bevorzugt zwischen 1,2 und 5 MDa aufweist.

3. Zusammensetzung gemäß irgendeinem der voranstehenden Ansprüche 1 oder 2, wobei die genannte Zusammensetzung eine flüssige Form aufweist und eine von 15 Pa.s bis 40 Pa.s reichende Viskosität, gemessen gemäß dem Brookfield-Testverfahren, bevorzugt von 18 Pa.a bis 32 Pa.s aufweist.

4. Zusammensetzung gemäß irgendeinem der voranstehenden Ansprüche, wobei die Hyaluronsäure als der Bestandteil (A) wenigstens teilweise zusammen mit dem Des(1-3)-IGF-I als ein analoger Säugetier-Insulinwachstumsfaktor-1 DVS vernetzt ist, der zwischen 1 und 5 Aminosäure-Reste aufweist, die bei der N-Endung fehlen.

5. Zusammensetzung zur Verwendung bei der Behandlung einer Krankheit, die die Gelenkknorpel beeinträchtigt, umfassend:
a) Hyaluronsäure als ein Bestandteil (A) oder Salze davon und / oder autologe Zellen, die zum Synthetisieren und Sekretieren eines Bestandteils (A) neu programmiert sind, und
b) Des(1-3)-IGF-I als ein analoger Säugetier-Insulinwachstumsfaktor-1, der 1 bis 5 Aminosäure-Reste aufweist, die bei der N-Endung fehlen.

6. Zusammensetzung zur Verwendung gemäß Anspruch 5, wobei die Menge an Hyaluronsäure als Bestandteil (A) in der genannten Zusammensetzung von 0,01 Gew.-% bis 5 Gew.-% basierend auf dem Gesamtgewicht der Zusammensetzung reicht.

7. Zusammensetzung zur Verwendung gemäß irgendeinem der voranstehenden Ansprüche 5 oder 6, wobei die Menge des Analogs größer ist als 10 µg/ml, bevorzugt größer als 30 µg/ml, weiter bevorzugt von 30 µg/ml bis 60 µg/l basierend auf dem Gesamtgewicht der Zusammensetzung reicht.

8. Zusammensetzung zur Verwendung gemäß irgendeinem der voranstehenden Ansprüche 5 bis 7, wobei die Hyaluronsäure als der Bestandteil (A) wenigstens teilweise zusammen mit dem Des(1-3)-IGF-I als ein analoger Säugetier-Wachstumsfaktor-1 DVS vernetzt ist, der 1 bis 5 Aminosäure-Reste aufweist, die bei der N-Endung fehlen.

9. Zusammensetzung zur Verwendung gemäß irgendeinem der voranstehenden Ansprüche 5 bis 8, die im ersten Monat der Behandlung gemeinsam ein bis vier Mal und 6 Monate danach einmal verabreicht wird.

10. Zusammensetzung gemäß irgendeinem der voranstehenden Ansprüche 5 bis 9 zur Verwendung bei einem Verfahren zum Begrenzen der Zerlegung der Hyaluronsäure und Verlängern des Viskosupplementations-Effekts von intraartikulären Injektionen der Hyaluronsäure in das Gelenk eines Menschen oder eines Tiers, umfassend die Schritte der Verabreichung der Zusammensetzung.

11. Verwendung des Des(1-3)-IGF-I als ein Analog des Säugetier-Insulinwachstumsfaktors-1, wobei 1 bis 5 Aminosäure-Reste von der N-Endung fehlen, um die Stabilität in einer flüssigen Zusammensetzung der Hyaluronsäure oder Salze davon zu erhöhen.

12. Prozess zur Zubereitung einer Zusammensetzung gemäß irgendeinem der voranstehenden Ansprüche 1 bis 4, umfassend die Schritte:
a) Reagieren von Divinylsulfon mit Hyaluronsäure als der Bestandteil (A) mit einem ph, der von 8 bis 11 reicht, unter Verwendung eines Divinylsulphons mit einem Molarverhältnis zwischen 1 und 10 % für den Bestandteil (A) ;
b) Hinzufügen des Des(1-3)-IGF-I als ein Analog des Säugetier-Insulinwachstumsfaktors-1, wobei die Aminosäure-Reste modifiziert, entfernt oder hinzugefügt werden, die die Rezeptor-Avidität vom Typ 1 des insulinartigen Wachstumsfaktors und die Affinität aufrecht erhalten oder verstärken, und wobei die Aminosäure-Reste modifiziert, entfernt oder hinzugefügt werden, die die Avidität und Affinität des den insulinartigen Wachstumsfaktor bindenden Proteins reduzieren.

13. Prozess gemäß dem voranstehenden Anspruch, wobei die Schritte (a) und (b) gleichzeitig bei einem von 8 bis 9 reichenden ph ausgeführt werden.

## Revendications

1. Composition comprenant :
a) de l'acide hyaluronique en tant que composé (A) ou sels dudit acide hyaluronique, et/ou des cellules autologues reprogrammées pour synthétiser et secréter un composé (A), et
b) une hormone des(1-3)-IGF-1 en tant qu'analogue du facteur de croissance insulinoïde 1 chez les mammifères, ledit analogue ayant de 1 à 5 résidus d'acides aminés absents de la terminaison N.

2. Composition selon la revendication précédente, dans laquelle l'acide hyaluronique, en tant que composé (A), est un composé à liaisons croisées, ayant un poids moléculaire compris entre 0,7 MDa et 8 MDa, de préférence compris entre 1,2 MDa et 5 MDa.

3. Composition selon l'une des revendications précédentes 1 ou 2, où ladite composition est sous une forme liquide et a une viscosité allant de 15 Pa.s à 40 Pa.s, ladite viscosité, telle qu'elle est mesurée selon la méthode d'essai de Brookfield, étant comprise de préférence entre 18 Pa.s et 32 Pa.s.

4. Composition selon l'une quelconque des revendications précédentes, dans laquelle l'acide hyaluronique en tant que composé (A) est au moins partiellement du divinylsulfone - DVS - ayant des liaisons croisées conjointement avec l'hormone des(1-3)-IGF-1 en tant qu'analogue du facteur de croissance insulinoïde 1 chez les mammifères, ledit analogue ayant de 1 à 5 résidus d'acides aminés absents de la terminaison N.

5. Composition pour un usage dans le traitement d'une maladie affectant le cartilage articulaire, ladite composition comprenant :
(a) de l'acide hyaluronique en tant que composé (A) ou sels dudit acide hyaluronique, et/ou des cellules autologues reprogrammées pour synthétiser et secréter un composé (A), et
(b) une hormone des(1-3)-IGF-1 en tant qu'analogue du facteur de croissance insulinoïde 1 chez les mammifères, ledit analogue ayant de 1 à 5 résidus d'acides aminés absents de la terminaison N.

6. Composition pour un usage selon la revendication 5, où la quantité d'acide hyaluronique, en tant que composé (A) dans ladite composition, va de 0,01 % en poids à 5 % en poids, ladite quantité étant basée sur le poids total de la composition.

7. Composition pour un usage selon l'une des revendications précédentes 5 ou 6, où la quantité de l'analogue est supérieure à 10 µg/ml, de préférence est supérieure à 30 µg/ml, de façon plus préférable va de 30 µg/ml à 60 µg/ml, ladite quantité étant basée sur le poids total de la composition.

8. Composition pour un usage selon l'une quelconque des revendications précédentes 5 à 7, dans laquelle l'acide hyaluronique en tant que composé (A) est au moins partiellement du divinylsulfone - DVS - ayant des liaisons croisées conjointement avec l'hormone des(1-3)-IGF-1 en tant qu'analogue du facteur de croissance insulinoïde 1 chez les mammifères, ledit analogue ayant de 1 à 5 résidus d'acides aminés absents de la terminaison N.

9. Composition pour un usage selon l'une quelconque des revendications précédentes 5 à 8, composition qui est administrée dans une articulation une à quatre fois au cours du premier mois de traitement et administrée une fois tous les 6 mois qui suivent.

10. Composition selon l'une quelconque des revendications précédentes 5 à 9, pour un usage dans un procédé permettant de limiter la dégradation de l'acide hyaluronique et de prolonger l'effet de viscosupplémentation d'injections intra-articulaires d'acide hyaluronique effectuées dans l'articulation d'un humain ou d'un animal, lesdites injections comprenant les étapes d'administration de la composition.

11. Usage de l'hormone des(1-3)-IGF-1 en tant qu'analogue du facteur de croissance insulinoïde 1 chez les mammifères, analogue dans lequel de 1 à 5 résidus d'acides aminés sont absents de la terminaison N, afin d'augmenter la stabilité dans une composition liquide d'acide hyaluronique ou de sels dudit acide hyaluronique.

12. Processus pour la préparation d'une composition selon l'une quelconque des revendications précédentes 1 à 4, ledit processus comprenant les étapes consistant :
(a) à faire réagir du divinylsulfone avec de l'acide hyaluronique en tant que composé (A), à un pH variant entre 8 et 11, en utilisant un rapport molaire du divinylsulfone, relativement au composé (A), compris entre 1 % et 10 % ;
(b) à ajouter une hormone des(1-3)-IGF-1 en tant qu'analogue du facteur de croissance insulinoïde 1 chez les mammifères, analogue dans lequel des résidus d'acides aminés sont modifiés, enlevés ou ajoutés, et qui maintiennent ou stimulent l'avidité et l'affinité du récepteur de type 1 du facteur de croissance analogue à l'insuline, et analogue dans lequel des résidus d'acides aminés sont modifiés, enlevés ou ajoutés, et qui réduisent l'avidité et l'affinité des protéines de liaison au facteur de croissance analogue à l'insuline.

13. Processus selon la revendication précédente, dans lequel les étapes (a) et (b) sont effectuées simultanément, à un pH variant entre 8 et 9.


### SEQUENCE LISTING

<110> NEWVEN sprl Stoffel, Bernard Humphrys, Simon Troy van der Tuin, Everhardus Sipsma, Charles Henrij
<120> Composition for the protection of polysaccharides in joints
<130> T573WO
<160> 5
<170> PatentIn version 3.5
<210> 1
   <211> 29
   <212> PRT
   <213> Escherichia coli
<400> 1
   Pro Glu Thr Leu Cys Gly Ala Glu Leu Val Asp Ala Leu Gln Phe Val
   1               5                   10                  15
   Cys Gly Asp Arg Gly Phe Tyr Phe Asn Lys Pro Thr Gly
               20                  25
<210> 2
   <211> 28
   <212> PRT
   <213> Escherichia coli
<400> 2
   Glu Thr Leu Cys Gly Ala Glu Leu Val Asp Ala Leu Gln Phe Val Cys
   1               5                   10                  15
   Gly Asp Arg Gly Phe Tyr Phe Asn Lys Pro Thr Gly
               20                  25
<210> 3
   <211> 27
   <212> PRT
   <213> Escherichia coli
<400> 3
   Thr Leu Cys Gly Ala Glu Leu Val Asp Ala Leu Gln Phe Val Cys Gly
   1               5                   10                  15
   Asp Arg Gly Phe Tyr Phe Asn Lys Pro Thr Gly
               20                  25
<210> 4
   <211> 26
   <212> PRT
   <213> Escherichia coli
<400> 4
   Leu Cys Gly Ala Glu Leu Val Asp Ala Leu Gln Phe Val Cys Gly Asp
   1               5                   10                  15
   Arg Gly Phe Tyr Phe Asn Lys Pro Thr Gly
               20                  25
<210> 5
   <211> 25
   <212> PRT
   <213> Escherichia coli
<400> 5
   Cys Gly Ala Glu Leu Val Asp Ala Leu Gln Phe Val Cys Gly Asp Arg
   1               5                   10                  15
   Gly Phe Tyr Phe Asn Lys Pro Thr Gly
               20                  25